# EUROPEAN PATENT APPLICATION

(11) **EP 4 331 563 A1**
(43) Date of publication of application: **06.03.2024**
(21) Application number: 22193038.1
(22) Date of filing: 31.08.2022
(51) Int. Cl.: A61K 8/04, A61K 8/06, A61K 8/21, A61K 8/25, A61K 8/27, A61K 8/29, A61K 8/34, A61K 8/46, A61K 8/73, A61P 1/02, A61P 1/04, A61Q 11/00, A61K 8/02, A61K 33/30

(54) **USE OF ORAL CARE COMPOSITION**

(71) Applicant: Unilever IP Holdings B.V., 3013 AL Rotterdam (NL)
(72) Inventor: ADAMS, Suzanne Elizabeth, 6708 WH Wageningen (NL); CAWLEY, Andrew Kenneth, 6708 WH Wageningen (NL); HUNT, Joanne Elizabeth, 6708 WH Wageningen (NL); SLOMKA, Vera, 6708 WH Wageningen (NL)
(74) Representative: Tansley, Sally Elizabeth

(57) **Abstract**

An oral composition comprising a zinc salt for use in a method for the enhancement of vitamin K2 levels within the mouth.

## Description

The present invention relates to oral care compositions, in particular to oral care compositions comprising zinc.

### Background

Zinc salts have been used in some oral care compounds for a number of years.

US 5 470 561 discloses an anti-plaque mouthwash comprising a zinc salt and triclosan. The composition may also comprise glycine and has a pH of between 4 and 8, preferably between 5 and 7, the preferred pH being 6.

Toothpaste compositions comprising zinc are disclosed in WO0612977. The toothpaste comprises water, a zinc salt, a chelating agent for the zinc.

Vitamin K2 seems to be of importance for oral and systemic health. Vitamin K2 is associated with the inhibition of arterial calcification and arterial stiffening. Further, Vitamin K2 activates osteocalcin, one of the main regulation proteins in dental health. Osteocalcin triggers a mechanism that stimulates the growth of new dentin (Koshihara Y, Hoshi K. Vitamin K2 enhances osteocalcin accumulation in the extracellular matrix of human osteoblasts in vitro. J Bone Miner Res. 1997 Mar;12(3):431-8. doi: 10.1359/jbmr.1997.12.3.431. PMID: 9076586.).

The present application has found that the presence of zinc in an oral care composition can deliver a benefit to the body in addition to oral care benefits..

### Description of the Invention

The present invention relates to an oral composition comprising a zinc salt for use in a method for the enhancement of vitamin K2 within the mouth.

A further aspect of the invention relates to an oral care composition comprising a zinc salt for use in activation of osteocalcin and the stimulation of the growth of new dentin.

For the avoidance of doubt, any feature of one aspect of the present invention may be utilised in any other aspect of the invention. Any feature described as 'preferred' should be understood to be particularly preferred in combination with a further preferred feature or features. Herein, any feature of a particular embodiment may be utilized in any other embodiment of the invention. The word 'comprising' is intended to mean 'including' but not necessarily 'consisting of or 'composed of. In other words, the listed steps or options need not be exhaustive. The examples given in the description below are intended to clarify the invention but not to limit the invention. All percentages are weight/weight percentages unless otherwise indicated.

### Detailed Description of the Invention

The present invention relates to oral compositions comprising a zinc salt to promotes enhancement of vitamin K2 by increasing the expression of genes associated with vitamin K2. Preferably the genes associated with vitamin K2 comprise: 2-succinyl-6-hydroxy-2,4-cyclohexadiene-1-carboxylate synthase EC:4.2.99.20, o-succinylbenzoate synthase EC:4.2.1.113, demethylmenaquinone methyltransferase EC:2.1.1.163 and NAD(P)H dehydrogenase EC:1.6.5.2 or mixtures thereof.

Biosynthesis of vitamin K2 by the oral microbiome positively contributes to the promotion of oral and systemic healthCompositions according to the invention comprise a zinc salt, preferably they comprise a water-soluble or sparingly water-soluble sources of zinc ions such as zinc chloride, zinc acetate, zinc gluconate, zinc sulphate, zinc fluoride, zinc lactate, zinc oxide, zinc monoglycerolate, zinc tartrate, zinc pyrophosphate and zinc maleate. Zinc citrate trihydrate is particularly preferred.

The zinc salt is preferably present at from 0.01 to 5% by weight of the total composition, more preferably 0.1 to 4 wt%, most preferably from 0.5 to 3.0wt%.

The composition of the invention is preferably used to clean the surfaces of the oral cavity and is known as an oral care composition, the composition may be suitable for cosmetic and/or medical use.

Accordingly, preferred product forms for compositions of the invention are those which are suitable for brushing and/or rinsing the surfaces of the oral cavity.

The composition of the invention is most preferably in the form of a dentifrice. The term "dentifrice" denotes an oral composition which is used to clean the surfaces of the oral cavity. Such a composition is not intentionally swallowed for purposes of systemic administration of therapeutic agents, but is applied to the oral cavity, used to treat the oral cavity and then expectorated. Typically, such a composition is used in conjunction with a cleaning implement such as a toothbrush, usually by applying it to the bristles of the toothbrush and then brushing the accessible surfaces of the oral cavity.

Preferably the dentifrice/toothpaste is in the form of an extrudable semi-solid such as a cream, paste or gel (or mixture thereof).

A composition according to the invention (such as a dentifrice/toothpaste) will generally contain further ingredients to enhance performance and/or consumer acceptability, in addition to the ingredients specified above.

Compositions according to the invention, particularly toothpastes, preferably comprise particulate abrasive materials such as silicas, aluminas, calcium carbonates, dicalciumphosphates, calcium pyrophosphates, hydroxyapatites, trimetaphosphates, insoluble hexametaphosphates and so on, including agglomerated particulate abrasive materials, usually in amounts between 3 and 60% by weight of the oral care composition.

Preferably the composition, particularly a toothpaste, comprises a silica based abrasive. The preferred abrasive silicas used in the present invention is a silica with a low refractive index. It may be used as the sole abrasive silica, or in conjunction with a low level of other abrasive silicas, e.g. those according to EP 236070. Typical examples of suitable low refractive index abrasive silicas (e.g. having an R.I. of between 1.435 and 1.445) are Tixosil 63 and 73 ex Rhone Poulenc; Sident 10 ex Degussa; Zeodent 113 ex Zeofinn; Zeodent 124 ex Huber, Sorbosil AC 77 ex Crosfield Chemicals (having an R.I. of approximately 1.440). The amount of these silicas in the total composition generally ranges from 5-60% by weight, usually 5-20% by weight.

The composition, particularly if a toothpaste, preferably comprises an inorganic or a natural or synthetic thickener or gelling agent in proportions of about 0.10 to about 15% by weight depending on the material chosen. These proportions of thickeners in the dentifrice compositions of the present invention form an extrudable, shape-retaining product which can be squeezed from a tube onto a toothbrush and will not fall between the bristles of the brush but rather, will substantially maintain its shape thereon. Suitable thickeners or gelling agents useful in the practice of the present invention include inorganic thickening silicas such as amorphous silicas available from Huber Corporation under the trade designation Zeodent 165, Irish moss, iota-carrageenan, gum tragacanth, and polyvinylpyrrolidone.

Compositions according to the invention preferably comprise a polymeric deposition aid. Preferably the composition comprises acid anhydride polymers, particularly preferred are co-polymers of maleic anhydride with methyl vinylether, in which the anhydride moiety may be in a partially or fully hydrolysed or alcoholysed form. Preferred copolymers include Gantrez(R) polymers such as:
Gantrez S-95: molecular weight 216,000; free acid;
Gantrez S-96: molecular weight 700,000; free acid;
Gantrez S-97: molecular weight 1,500,000; free acid; and
Gantrez MS-955: molecular weight 1,060,000; calcium/sodium salt.

Particularly preferred co-polymers of maleic acid and methyl vinylether have a molecular weight of 1,000,000 or greater and an especially preferred material is Gantrez S-97.

Compositions according to the invention may comprise a tooth whitening agent. The whitening agent preferably comprises a green and/or a blue pigment. In the context of the present invention a pigment is generally understood to be a shade/material which is insoluble in the relevant medium, at the relevant temperature. This is in contrast to dyes which are soluble. In the context of this invention, the "relevant medium" is human saliva, the liquid medium in which the composition is used, at the temperature of the oral cavity during brushing of the teeth, i.e. up to 37 Degrees C. As a reasonable approximation, the relevant medium may be considered to be water and the relevant temperature to be 25 Degrees C.

Preferably the blue pigment is Pigment Blue 15, more preferably Pigment Blue 15:1, 15:2, 15:3, 15:4, 15:5 or 15:6, most preferably 15:1. A preferred pigment is blue pigment is Phthalocyanine Blue Pigment, CI No. 74160, blue covarine.

The preferred Green pigment is Phthalocyanine Green, preferably Phthalocyanine Green CI-74260.

Preferably the total level of pigment in the composition is from 0.01 wt% to 3 wt, more preferably from 0.02 to 2 wt%.

If the composition is a toothpaste it may be a dual phase paste, with the whitening pigments present in one phase.

Compositions according to the invention may comprise oral care enzyme systems such as hydrogen peroxide producing enzyme systems (e.g. the oxidoreductase enzyme glucose oxidase), amyloglucosidase, dextranase and/or mutanase, (optionally in the presence of zinc ion providing compounds and/or 8- hydroxyquinoline derivatives), lactoperoxidase, lactoferrin, lysozyme and mixtures thereof.

Compositions of the invention may comprise fluoride sources such as sodium fluoride, stannous fluoride, sodium monofluorophosphate, zinc ammonium fluoride, tin ammonium fluoride, calcium fluoride, cobalt ammonium fluoride and mixtures thereof.

In one embodiment a preferred class of oral care active for inclusion in the compositions of the invention includes agents for the remineralisation of teeth. The term "remineralisation" in the context of the present invention means the *in situ* generation of hydroxyapatite on teeth.

A specific example of a suitable agent for the remineralisation of teeth is a mixture of a calcium source and a phosphate source which, when delivered to the teeth results in the *in situ* generation of hydroxyapatite on teeth.

Illustrative examples of the types of calcium source that may be used in this context (hereinafter termed "remineralising calcium sources") include, for example, calcium phosphate, calcium gluconate, calcium oxide, calcium lactate, calcium glycerophosphate, calcium carbonate, calcium hydroxide, calcium sulphate, calcium carboxymethyl cellulose, calcium alginate, calcium salts of citric acid, calcium silicate and mixtures thereof. Preferably the remineralising calcium source is calcium silicate.

The amount of remineralising calcium source(s) (e.g. calcium silicate) in the composition of the invention typically ranges from 1 to 30%, preferably from 5 to 20% by total weight remineralising calcium source based on the total weight of the oral care composition.

Illustrative examples of the types of phosphate source that may be used in this context (hereinafter termed "remineralising phosphate sources") include, for example, monosodium dihydrogen phosphate, disodium hydrogen phosphate, sodium pyrophosphate, tetrasodium pyrophosphate, sodium tripolyphosphate, sodium hexametaphosphate, potassium dihydrogenphosphate, trisodium phosphate, tripotassium phosphate and mixtures thereof. Preferably the remineralising phosphate source is a mixture of trisodium phosphate and sodium dihydrogen phosphate.

The amount of remineralising phosphate source(s) (e.g. trisodium phosphate and sodium dihydrogen phosphate) in the composition of this invention typically ranges from 2 to 15%, preferably from 4 to 10% by total weight remineralising phosphate source based on the total weight of the oral care composition.

Mixtures of any of the above described materials may also be used.

The composition according the invention will comprise further ingredients which are common in the art, such as:
antimicrobial agents, e.g. chlorhexidine, sanguinarine extract, metronidazole, quaternary ammonium compounds, such as cetylpyridinium chloride; cetylpyridium chloride clay complex, bis-guanides, such as chlorhexidine digluconate, hexetidine, octenidine, alexidine; and halogenated bisphenolic compounds, such as 2,2' methylenebis-(4-chloro-6-bromophenol);
anti-inflammatory agents such as ibuprofen, flurbiprofen, aspirin, indomethacin etc.;
anti-caries agents such as sodium- and stannous fluoride, aminefluorides, sodium monofluorophosphate, sodium trimeta phosphate and casein;
plaque buffers such as urea, calcium lactate, calcium glycerophosphate and strontium polyacrylates;
vitamins such as Vitamins A, C and E;
plant extracts;
plant-derivable antioxidants such as flavonoid, catechin, polyphenol, and tannin compounds and mixtures thereof;
desensitising agents, e.g. potassium citrate, potassium chloride, potassium tartrate, potassium bicarbonate, potassium oxalate, potassium nitrate and strontium salts;
anti-calculus agents, e.g. alkali-metal pyrophosphates, hypophosphite-containing polymers, organic phosphonates and phosphocitrates etc.;
biomolecules, e.g. bacteriocins, antibodies, enzymes, etc.;
flavours, e.g. peppermint and spearmint oils;
proteinaceous materials such as collagen;
preservatives;
opacifying agents;
amino acids such as arginine;
colouring agents;
pH-adjusting agents;
sweetening agents;
pharmaceutically acceptable carriers, e.g. starch, sucrose, water or water/alcohol systems etc.;
surfactants, such as anionic, nonionic, cationic and zwitterionic or amphoteric surfactants;
Humectants such as glycerol, sorbitol, propyleneglycol, xylitol, lactitol etc.;
binders and thickeners such as sodium carboxymethyl-cellulose, hydroxyethyl cellulose (Natrosol^{®}), xanthan gum, gum arabic etc. as well as synthetic polymers such as polyacrylates and carboxyvinyl polymers such as Carbopol^{®};
polymeric compounds which can enhance the delivery of active ingredients such as antimicrobial agents can also be included;
buffers and salts to buffer the pH and ionic strength of the oral care composition; and other optional ingredients that may be included are e.g. bleaching agents such as peroxy compounds e.g. potassium peroxydiphosphate, effervescing systems such as sodium bicarbonate/citric acid systems, colour change systems, and so on.

Examples will now be illustrated by the following non-limiting Examples:

### Examples

A clinical study was conducted to determine the effect of use of a product containing zinc citrate trihydrate compared to a control product on the oral microbiome as shown in Table 1.

**Table 1**

| **Ingredient** | **Example A wt%** | **Example** 1 **wt%** | **Wash out product** |
|---|---|---|---|
| Sorbitol | 45 | 45 | 45 |
| Hydrated silica | 18 | 18 | 17.5 |
| PEG32 | 2 | 2 | 5 |
| Sodium Lauryl sulphate | 1.8 | 1.8 | 1.5 |
| Titanium dioxide | 1 | 1 | 1 |
| Carboxy methyl cellulose | 0.8 | 0.8 | 0.63 |
| Sodium Fluoride | 0.32 | 0.32 | 0.32 |
| Zinc Citrate Trihydrate | 0 | 2 | 0 |
| Water and minors | To 100 | To 100 | To 100 |

A clinical study was conducted to determine the effect of use of a product containing 2% zinc citrate trihydrate compared to a control product on the oral microbiome. Following a 4-week washout period subjects used one of the two test products for a 6-week period with samples of dental plaque collected at baseline, 2 weeks and 6 weeks. The samples were collected into an RNA protect solution to stabilise the RNA prior to analysis of microbiome gene activity (transcriptomics).

### RNA extraction and sequencing

Plaque samples were pooled for each product and time point and spilt into three to provide replicates for RNA extraction and analysis, in total yielding 18 samples. The RNA was extracted using the Qiagen AllPrep kit following the manufactures instructions and quantified using Qubit HS RNA kit again following the manufactures instructions. The RNA samples were then sent to the Centre for Genomic Research at the University of Liverpool for RNA sequencing. Following depletion of the ribosomal RNA (rRNA) to allow sequencing of only the messenger RNA (mRNA) to focus on the more informative parts of the transcriptome, libraries were sequenced on an Illumina HiSeq 4000 platform.

### Gene expression analysis

RNA sequence data was processed using a bioinformatics pipeline (Eagle Genomics) to determine microbiome community activity via analysis of gene expression (mRNA) using the Human2 database. Analysis of individual genes was conducted using JMP.

Analysis of metabolic pathways associated with vitamin K2 biosynthesis and changes with the two test products were conducted as described above.

A significant increase (t-test, p value < 0.05) in a group, consisting of 4 genes associated with Vitamin K2 biosynthesis was observed for the zinc product compared to the control product at 6 weeks of product use, whilst no significant product difference was observed at baseline (table 1). At week 6, there was significant increases for the zinc product compared to the control product.

Table 2: Relative abundance (%) of genes associated with vitamin K2 biosynthesis (f(2-succinyl-6-hydroxy-2,4-cyclohexadiene-1-carboxylate synthase EC:4.2.99.20, o-succinylbenzoate synthase EC:4.2.1.113, demethylmenaquinone methyltransferase EC:2.1.1.163 and NAD(P)H dehydrogenase EC:1.6.5.2) or the Control and Zinc pastes at different timepoints (mean (std dev)).

**Table 2**

| | **Initial measurement** | **Week 6** | **Baseline - Week 6** |
|---|---|---|---|
| Example A | 0.026 (0.004) | 0.024 (0.001) | p = 0.513 |
| Example 1 | 0.027(0.013) | 0.033 (0.002) | p = 0.033 |
| | p = 0.310 | p = 0.002 | |

The results show use of a zinc containing toothpaste significantly increases the expression of genes involved in the production of vitamin K2 compared to a control toothpaste.

## Claims

1. An oral composition comprising a zinc salt for use in a method for the enhancement of vitamin K2 levels within the mouth.

2. An oral care composition according claim 1 in which the composition promotes enhancement of vitamin K2 by increasing the expression of genes associated with vitamin K2.

3. An oral care composition according to claim 2 in which the genes associated with vitamin K2 are selected from the group consisting of 2-succinyl-6-hydroxy-2,4-cyclohexadiene-1-carboxylate synthase EC:4.2.99.20, o-succinylbenzoate synthase EC:4.2.1.113, demethylmenaquinone methyltransferase EC:2.1.1.163 and NAD(P)H dehydrogenase EC:1.6.5.2)or mixtures thereof.

4. An oral care composition for use according to any preceding claim in which the zinc salt is zinc citrate trihydrate.

5. An oral care composition for use according to any preceding claim in which the level of zinc salt is from 0.1 to 5 wt% of the total composition.

6. An oral care composition for use according to any preceding claim that further comprises silica.

7. An oral care composition for use according to any preceding claim that further comprises an anionic and/or nonionic cleansing surfactant.

8. A composition for use according to any preceding claim that is a dentifrice composition.

9. An oral care composition comprising a zinc salt for use an oral care composition comprising a zinc salt for use in activation of osteocalcin and the stimulation of the growth of new dentin.
